(19) Europäisches Patentamt / European Patent Office / Office européen des brevets

(11) **EP 4 438 074 B1**

(12) **EUROPÄISCHE PATENTSCHRIFT**

(45) Veröffentlichungstag und Bekanntmachung des Hinweises auf die Patenterteilung:
**28.05.2025 Patentblatt 2025/22**

(21) Anmeldenummer: **24166697.3**

(22) Anmeldetag: **27.03.2024**

(51) Internationale Patentklassifikation (IPC):
**A61M 1/36** (2006.01)

(52) Gemeinsame Patentklassifikation (CPC):
**A61M 1/3656;** A61M 2205/18; A61M 2205/3334;
A61M 2205/50; A61M 2230/207; A61M 2230/30

(54) **VERFAHREN UND SYSTEM ZUR BETRIEBSÜBERWACHUNG EINES MEDIZINISCHEN GERÄTS ZUR EXTRAKORPORALEN BLUTBEHANDLUNG**

METHOD AND SYSTEM FOR MONITORING THE OPERATION OF A MEDICAL DEVICE FOR EXTRACORPOREAL BLOOD TREATMENT

PROCÉDÉ ET SYSTÈME DE SURVEILLANCE DU FONCTIONNEMENT D'UN APPAREIL MÉDICAL POUR LE TRAITEMENT EXTRACORPOREL DU SANG

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **31.03.2023 DE 102023108353**

(43) Veröffentlichungstag der Anmeldung:
**02.10.2024 Patentblatt 2024/40**

(73) Patentinhaber: **B. Braun Avitum AG**
**34212 Melsungen (DE)**

(72) Erfinder:
• **Wagner, Andre**
**34117 Kassel (DE)**
• **Mueglich, Nicolas David**
**34233 Fuldatal (DE)**

(74) Vertreter: **Patentanwälte**
**Ruff, Wilhelm, Beier, Dauster & Partner mbB**
**Kronenstraße 30**
**70174 Stuttgart (DE)**

(56) Entgegenhaltungen:
EP-A1- 2 913 072      EP-A2- 1 938 847
WO-A1-2011/098243     US-A1- 2010 073 171

**Beschreibung**

Die Erfindung betrifft ein Verfahren zum Überwachen des Betriebs eines medizinischen Geräts zur extrakorporalen Blutbehandlung, ein Überwachungssystem für ein medizinisches Gerät zur extrakorporalen Blutbehandlung und ein medizinisches Gerät zur extrakorporalen Blutbehandlung mit einem solchen Überwachungssystem.

Extrakorporale Blutbehandlungen finden üblicherweise im Rahmen einer Nierenersatztherapie statt, beispielsweise in Form einer Hämodialyse, Hämofiltration oder Hämodiafiltration.

Übliche Geräte zur extrakorporalen Blutbehandlung weisen eine Blutbehandlungseinheit mit einer Blutkammer, einer Behandlungskammer und einer zwischen den beiden Kammern angeordneten semipermeablen Membran auf. Im Betrieb des Geräts wird die Blutkammer vom Blut eines Patienten durchströmt. Die Behandlungskammer wird von einer Behandlungsflüssigkeit durchströmt. Harnpflichtige Flüssigkeit wird über die semipermeable Membran aus dem durch die Blutkammer strömenden Blut entfernt und mit der Behandlungsflüssigkeit abtransportiert. Die dem Blut hierbei entzogene Flüssigkeitsmenge pro Zeiteinheit wird als Ultrafiltrationsrate oder Filtrationsvolumenstrom bezeichnet. Der Filtrationsvolumenstrom kann an dem medizinischen Gerät eingestellt werden, beispielsweise über die Steuerung einer die Behandlungsflüssigkeit fördernden Filtrationspumpe. Zur Leitung des Bluts zwischen dem Patienten und der Blutbehandlungseinheit weisen übliche medizinische Geräte eine arterielle Fluidleitung und eine venöse Fluidleitung auf. Die arterielle Fluidleitung ist einends an einen Einlass der Blutkammer angeschlossen und andernends zur Verbindung mit einem arteriellen Gefäßzugang des Patienten eingerichtet. Die venöse Fluidleitung ist einends an einen Auslass der Blutkammer angeschlossen und andernends zur fluidleitenden Verbindung mit einem venösen Gefäßzugang des Patienten eingerichtet. Die blutführenden Komponenten des medizinischen Geräts, insbesondere die Blutkammer sowie die besagten Fluidleitungen, werden auch als extrakorporaler Blutkreislauf bezeichnet. Übliche medizinische Geräte weisen eine Pumpe zum Fördern des Bluts entlang des extrakorporalen Blutkreislaufs auf. Der Pumpvolumenstrom der Pumpe ist einstellbar und bewegt sich üblicherweise im Bereich mehrerer Hundert Milliliter pro Minute. Die Verbindung des extrakorporalen Blutkreislaufs mit dem Blutkreislauf des Patienten erfolgt üblicherweise über entsprechende Kanülen oder Katheter, die jeweils andernends an den besagten Fluidleitungen angebracht/anbringbar sind und mit welchen eine arteriovenöse Fistel oder ein Gefäßimplantat des Patienten punktiert werden kann.

Störungen oder gar Unterbrechungen des Blutflusses in dem extrakorporalen Blutkreislauf und/oder dessen Verbindung mit dem Blutkreislauf können den Patienten gefährden.

Aus dem Stand der Technik sind deshalb Verfahren und Systeme zur diesbezüglichen Betriebsüberwachung medizinischer Geräte zur extrakorporalen Blutbehandlung bekannt.

Aus der EP 2 913 072 A1 ist ein System und ein Verfahren zur Überwachung eines Dialysegeräts bekannt, bei dem eine venöse Nadeldiskonnektion über eine Auswertung der in den Fluidleitungen vorherrschenden Fluiddrücke erkannt werden soll.

Weiter ist aus der EP 1 938 847 A2 ein Verfahren zur Betriebsüberwachung eines Dialysegeräts bekannt, bei welchem eine elektrische Leitfähigkeit der Behandlungsflüssigkeit ausgewertet wird.

Aufgabe der Erfindung ist es, ein Verfahren, ein Überwachungssystem und ein medizinisches Gerät der eingangs genannten Art bereitzustellen, die jeweils Vorteile gegenüber dem Stand der Technik bieten.

Diese Aufgabe wird durch das Bereitstellen eines Verfahrens mit den Merkmalen des Anspruchs 1, eines Überwachungssystems mit den Merkmalen des Anspruchs 10 und eines medizinischen Geräts mit den Merkmalen des Anspruchs 14 gelöst. Vorteilhafte Ausgestaltungen sind Gegenstand der Unteransprüche.

Das erfindungsgemäße Verfahren ist zum Überwachen des Betriebs eines medizinischen Geräts zur extrakorporalen Blutbehandlung vorgesehen. Das zu überwachende medizinische Gerät weist eine Blutbehandlungseinheit, eine arterielle Fluidleitung, eine venöse Fluidleitung und eine Blutpumpe auf. Die Blutbehandlungseinheit weist eine Blutkammer, die während des Betriebs von Blut durchströmt wird, und eine Behandlungskammer auf, die während des Betriebs von einer Blutbehandlungsflüssigkeit durchströmt wird und über eine semipermeable Membran von der Blutkammer getrennt ist. Die arterielle Fluidleitung ist einends mit einem Einlass der Blutkammer und andernends mit einem arteriellen Gefäßzugang eines Patienten fluidleitend verbunden und wird von einem arteriellen Volumenstrom durchströmt wird. Die venöse Fluidleitung ist einends mit einem Auslass der Blutkammer und andernends mit einem venösen Gefäßzugang des Patienten fluidleitend verbunden und wird von einem venösen Volumenstrom durchströmt. Die Blutpumpe ist zum Fördern eines Pumpvolumenstroms durch einen extrakorporalen Blutkreislauf des medizinischen Geräts eingerichtet. Der extrakorporale Blutkreislauf umfasst die Blutkammer, die arterielle Fluidleitung und die venöse Fluidleitung.

Dabei weist das Verfahren die Schritte auf: Ermitteln eines Kontrollparameters, der ein Verhältnis zwischen dem venösen Volumenstrom durch die venöse Fluidleitung und dem arteriellen Volumenstrom durch die arterielle Fluidleitung repräsentiert; Überwachen des Betriebs in Abhängigkeit des ermittelten Kontrollparameters.

Das erfindungsgemäße Verfahren erlaubt eine Betriebsüberwachung auf Grundlage eines rechnerischen Vergleichs zwischen dem venösen Volumenstrom in der venösen Fluidleitung und dem arteriellen Volumenstrom in der arteriellen Fluidleitung. Der besagte rechnerische Vergleich wird durch das Verhältnis, d.h. einen Quotienten, zwischen den beiden Volumenströmen und damit den Kontrollparametern gebildet. Der Kontrollparameter stellt einen quantitativen Vergleichswert zwischen dem venösen Volumenstrom und dem arteriellen Volumenstrom dar. Der Kontrollparameter als solcher, eine zeitliche Änderung des Kontrollparameters und/oder ein Vergleich des Kontrollparameters mit einem Referenzparameter erlauben einen Rückschluss auf eine Störung des Blutflusses in dem extrakorporalen Blutkreislauf und/oder dessen Verbindung mit dem Blutkreislauf des Patienten. Die Ermittlung des Kontrollparameters erfolgt bei unterschiedlichen Ausgestaltungen auf unterschiedliche Weise. Bei einer Ausgestaltung erfolgt eine direkte messtechnische Erfassung der beiden Volumenströme mittels hierfür geeigneter Sensoren. Bei einer bevorzugten Ausgestaltung werden die Volumenströme und/oder deren Verhältnis, bevorzugt approximiert, berechnet. Die approximierte Berechnung erfolgt vorzugsweise auf der Grundlage von Bestimmungsgleichungen, die einen modellhaften und/oder näherungsweisen funktionalen Zusammenhang zwischen Betriebsparametern des medizinischen Geräts und/oder an dem medizinischen Gerät ermittelten Messgrößen und den Volumenströmen oder deren Verhältnis abbilden. Die Überwachung in Abhängigkeit des ermittelten Kontrollparameters erfolgt bei unterschiedlichen Ausgestaltungen auf unterschiedliche Weise. Bei einer Ausgestaltung umfasst die Überwachung eine grafische, textbasierte und/oder zeichenbasierte Anzeige des Kontrollparameters zur Wahrnehmung durch medizinisches Personal. Das medizinische Personal kann in Abhängigkeit der Anzeige Maßnahmen zur Steuerung oder Abschaltung des medizinischen Geräts ergreifen. Bei einer weiteren Ausgestaltung umfasst die Überwachung die Ermittlung eines Überwachungssignals in Abhängigkeit des Kontrollparameters. Das Überwachungssignal kann akustisch und/oder optisch zur Wahrnehmung durch medizinisches Personal ausgegeben werden. Alternativ oder zusätzlich kann das Überwachungssignal als Steuersignal für das medizinische Gerät dienen, beispielsweise um den Betrieb des medizinischen Geräts mit veränderten Betriebsparametern fortzusetzen und/oder zu beenden.

In Ausgestaltung der Erfindung umfasst das Verfahren die Schritte: Erfassen einer Änderung des Kontrollparameters über der Zeit; Überwachen des Betriebs in Abhängigkeit der erfassten zeitlichen Änderung des Kontrollparameters. Bei dieser Ausgestaltung wird der Kontrollparameter vorzugsweise kontinuierlich oder quasi-kontinuierlich über der Zeit erfasst. Das Überwachen in Abhängigkeit der zeitlichen Änderung des Kontrollparameters lässt frühzeitig erkennen, ob und in welchem Umfang das Verhältnis der Volumenströme sich ändert. Die zeitliche Änderung lässt Rückschlüsse auf eine sich abzeichnende Störung des Blutflusses in dem extrakorporalen Blutkreislauf und/oder dessen Verbindung mit dem Blutkreislauf des Patienten zu. Beispielsweise kann durch die Überwachung der zeitlichen Änderung des Kontrollparameters eine Änderung der Eigenschaften der Gefäßzugänge erkannt werden. Außerdem kann eine Nadeldiskonnektion erkannt werden, d.h. ein Lösen der Fluidleitungen von dem betreffenden Gefäßzugang. Davon ausgehend, dass der Kontrollparameter gemäß

$$K = \frac{Q_v}{Q_a}$$

gebildet wird, wobei K den Kontrollparameter, $Q_v$ den venösen Volumenstrom und $Q_a$ den arteriellen Volumenstrom bezeichnet, kann die Überwachung der zeitlichen Änderung des Kontrollparameters folgende Informationen liefern: Sofern der Kontrollparameter über der Zeit ansteigt, kann dies ein Indikator dafür sein, dass die venöse Fluidleitung beeinträchtigt ist, beispielsweise weil eine andernends der venösen Fluidleitung angeordnete venöse Nadel sich zusetzt. Sofern der Kontrollparameter über der Zeit sinkt, kann dies ein Indikator dafür sein, dass die arterielle Fluidleitung beeinträchtigt ist, beispielsweise weil eine arterielle Nadel sich zusetzt. Ein Sinken des Kontrollparameters über der Zeit kann zudem ein Indikator dafür sein, dass die venöse Fluidleitung von dem venösen Gefäßzugang getrennt ist ("venöse Nadeldiskonnektion"). Auf ähnliche Weise können ein Lösen der arteriellen Fluidleitung oder eine Veränderung der arteriovenösen Fistel erkannt werden.

In weiterer Ausgestaltung der Erfindung umfasst das Verfahren die Schritte: Ermitteln eines theoretischen Kontrollparameters, der ein theoretisches Verhältnis zwischen dem venösen Volumenstrom und dem arteriellen Volumenstrom repräsentiert, wobei der theoretische Kontrollparameter als Funktion des Pumpvolumenstroms und eines Filtrationsvolumenstroms, der über die semipermeable Membran aus der Blutkammer abgeleitet wird, ermittelt wird; Vergleichen des ermittelten Kontrollparameters mit dem ermittelten theoretischen Kontrollparameter; Überwachen des Betriebs in Abhängigkeit des Vergleichs. Im Idealfall erfährt der Kontrollparameter im Laufe des Betriebs des medizinischen Geräts, d.h. während der extrakorporalen Blutbehandlung, keine nennenswerte Veränderung. Eine Veränderung tritt allerdings zwangsläufig dann auf, wenn die über die semipermeable Membran aus dem Blutstrom abgeführte Ultrafiltrationsrate, d.h. der Filtrationsvolumenstrom, verändert wird. Eine Veränderung des Filtrationsvolumenstroms kann durch eine Einstellung an dem medizinischen Gerät vorgenommen werden. Um einer solchen veränderten Geräteeinstellung bei der Überwachung Rechnung zu tragen, sieht diese Ausgestaltung der Erfindung einen Vergleich des ermittelten Kontrollparameters mit dem besagten theoretischen Kontrollparameter vor. Der theoretische Kontrollparameter wird vorzugsweise gemäß der Gleichung

$$K_t = \frac{Q_p - Q_F}{Q_p}$$

gebildet. Dabei bezeichnet $K_t$ den theoretischen Kontrollparameter, $Q_p$ den Pumpvolumenstrom und $Q_F$ den Filtrationsvolumenstrom. Beide genannten Volumenstrome sind einstellbare Betriebsparameter des medizinischen Geräts. Durch das Vergleichen des Kontrollparameters mit dem theoretischen Kontrollparameter kann vermieden werden, dass eine veränderte Einstellung des Filtrationsvolumenstroms die Überwachung beeinflusst.

In weiterer Ausgestaltung der Erfindung werden der arterielle Volumenstrom und der venöse Volumenstrom jeweils unmittelbar messtechnisch erfasst. Die messtechnische Erfassung erfolgt mittels hierfür geeigneter Sensoren.

In weiterer Ausgestaltung der Erfindung werden der arterielle Volumenstrom und der venöse Volumenstrom auf Grundlage der Hagen-Poiseuille-Gleichung approximiert ermittelt. Dies ist eine besonders bevorzugte Ausgestaltung der Erfindung. Die Hagen-Poiseuille-Gleichung beschreibt modellhaft vereinfacht den Volumenstrom einer laminaren stationären Strömung eines homogenen Newton'schen Fluids durch ein Rohr (Kapillar) mit einem Radius r und einer Länge l. Dabei ergibt sich der approximierte Volumenstrom Q gemäß der Gleichung

$$Q = \frac{\pi * r^4 * (\Delta P)}{8 * \eta * l}$$

wobei $\eta$ die dynamische Viskosität und $\Delta P$ den Druckverlust über der Länge l bezeichnen. Folglich gilt für den arteriellen Volumenstrom:

$$Q_a = \frac{\pi * r_a^4 * (\Delta P_a)}{8 * \eta_a * l_a}$$

Für den venösen Volumenstrom gilt dementsprechend:

$$Q_v = \frac{\pi * r_v^4 * (\Delta P_v)}{8 * \eta_v * l_v}$$

Der Kontrollparameter ergibt sich folglich gemäß

$$K = Q_v / Q_a = \frac{\eta_a * \Delta P_v}{\eta_v * \Delta P_a}$$

wobei vereinfachend angenommen wird, dass $l_v = l_a$ und $r_v = r_a$. Hierbei wird deutlich, dass durch die Ermittlung des Kontrollparameters, d.h. die Bildung des Quotienten aus den Volumenströmen, insbesondere der Radius und die Länge aus der Bestimmungsgleichung entfernt werden können. Die Erfinder haben erkannt, dass die Länge und der Radius Fertigungstoleranzen unterliegen. Eine direkte Verwendung dieser Größen für die approximierte Ermittlung der beiden Volumenströme würde deshalb unter Umständen zu Ungenauigkeiten führen. Dies umso mehr, als der Radius exponentiell in die Hagen-Poiseuille-Gleichung eingeht. Wird anstelle der Volumenströme deren Verhältnis (der Kontrollparameter) als Überwachungsgröße verwendet, können diese Ungenauigkeiten vermieden werden.

In weiterer Ausgestaltung des Verfahrens umfasst das approximierte Ermitteln: Ermitteln eines venösen Differenzdrucks zwischen der venösen Fluidleitung und dem venösen Gefäßzugang und eines arteriellen Differenzdrucks zwischen der arteriellen Fluidleitung und dem arteriellen Gefäßzugang; Ermitteln einer arteriellen Viskosität des arteriellen Volumenstroms und einer venösen Viskosität des venösen Volumenstroms; Berechnen des Kontrollparameters als Funktion der erfassten Differenzdrücke und der erfassten Viskositäten. Die besagten Differenzdrücke und Viskositäten werden der approximierten Ermittlung des Volumenstromverhältnisses auf Grundlage der Hagen-Poiseuille-Gleichung zugrunde gelegt. Die Differenzdrücke werden bei unterschiedlichen Ausgestaltungen auf unterschiedliche Weise ermittelt. Entsprechendes gilt sinngemäß für die Ermittlung der Viskositäten. Bei einer Ausgestaltung werden die Viskositäten unmittelbar messtechnisch mittels hierfür geeigneter Sensoren erfasst. Bei einer weiteren Ausgestaltung werden die Viskositäten auf der Grundlage physikalischer Modellgleichungen, vorzugsweise approximiert, berechnet. Für die Berechnung können an dem medizinischen Gerät eingestellte Betriebsparameter und/oder erfasste Messgrößen verwendet werden.

In weiterer Ausgestaltung der Erfindung umfasst das Ermitteln der Differenzdrücke: Messen eines venösen Fluiddrucks in der venösen Fluidleitung und eines arteriellen Fluiddrucks in der arteriellen Fluidleitung; Erfassen, insbesondere nicht-invasives Messen, eines mittleren arteriellen Drucks des Patienten. Der venöse Fluiddruck und der arterielle Fluiddruck werden vorzugsweise jeweils mittels eines hierfür geeigneten Sensors ermittelt. Die besagten Sensoren sind vorzugsweise an der betreffenden Fluidleitung angeordnet. Der mittlere arterielle Druck, abgekürzt oftmals MAD oder MAP (englisch: Mean Arterial Pressure), beschreibt den Mittelwert der Blutdruckkurve über der Zeit. Der mittlere arterielle Druck kann näherungsweise als in den Gefäßzugängen vorherrschender Fluiddruck angesetzt werden. Der mittlere arterielle Druck kann ermittelt werden gemäß

$$P_m = \frac{2}{3} * P_{sys} + \frac{1}{3} * P_{dias}$$

wobei $P_m$ den mittleren arteriellen Druck, $P_{sys}$ den systolischen und $P_{dias}$ den diastolischen Blutdruck bezeichnen. Der mittlere arterielle Druck wird vorzugsweise über eine nicht-invasive Blutdruckmessung an dem Patienten ermittelt. Alternativ kann der mittlere arterielle Druck im Rahmen des Verfahrens ohne vorherige Messung auf Grundlage von Erfahrungswerten, Datenbankwerten oder dergleichen erfasst werden. Eine Messung direkt am Patienten ist somit im Rahmen des Verfahrens folglich nicht unbedingt erforderlich. Der venöse Differenzdruck kann folglich auf Grundlage des gemessenen venösen Fluiddrucks und des erfassten mittleren arteriellen Drucks des Patienten wie folgt ermittelt werden:

$$\Delta P_v = P_v - P_m$$

Entsprechendes gilt sinngemäß für den arteriellen Differenzdruck, wobei

$$\Delta P_a = P_m - P_a$$

In weiterer Ausgestaltung der Erfindung umfasst das Ermitteln der Viskositäten: Messen eines arteriellen Hämatokritwerts des Bluts in der arteriellen Fluidleitung und/oder eines venösen Hämatokritwerts des Bluts in der venösen Fluidleitung; Ermitteln der arteriellen Viskosität als Funktion des gemessenen arteriellen Hämatokritwerts und/oder der venösen Viskosität als Funktion des gemessenen venösen Hämatokritwerts. Diese Ausgestaltung der Erfindung geht von der Überlegung aus, dass eine unmittelbare messtechnische Erfassung der Viskositäten vergleichsweise aufwändig ist und insbesondere Modifikationen an dem medizinischen Gerät erforderlich machen kann. Eine Ermittlung der Viskositäten auf Grundlage einer Messung der Hämatokritwerte bietet dementgegen besondere Vorteile. Denn übliche medizinische Geräte zur extrakorporalen Blutbehandlung sind oftmals bereits zur Messung des arteriellen und/oder venösen Hämatokritwerts eingerichtet. Zu diesem Zweck kann eine entsprechende Sensorik vorhanden sein. Weiter geht diese Ausgestaltung der Erfindung von der Überlegung aus, dass die Viskosität von Blut in einem näherungsweisen funktionalen Zusammenhang mit dem Hämatokritwert steht. Bei gemessenem Hämatokritwert kann folglich auf Grundlage der bekannten Korrelation/Funktion die Viskosität auf einfache Weise ermittelt werden. Der Zusammenhang ist näherungsweise linear und basiert auf empirischen Daten. Bei einer Ausgestaltung wird sowohl der arterielle als auch der venöse Hämatokritwert messtechnisch erfasst. Bei einer weiteren Ausgestaltung wird lediglich einer der beiden Hämatokritwerte messtechnisch erfasst, der weitere Hämatokritwert wird approximiert ermittelt.

In weiterer Ausgestaltung der Erfindung wird der arterielle Hämatokritwert messtechnisch erfasst und die venöse Viskosität wird approximiert ermittelt als Funktion des Pumpvolumenstroms, des Filtrationsvolumenstroms und des messtechnisch erfassten arteriellen Hämatokritwerts. Durch diese Ausgestaltung der Erfindung kann auf messtechnischen Aufwand zur Ermittlung beider Hämatokritwerte verzichtet werden. Lediglich einer der beiden Hämatokritwerte, nämlich der arterielle, wird gemessen. Der weitere, venöse Hämatokritwert wird rechnerisch ermittelt. Selbstverständlich ist auch eine umgekehrte Vorgehensweise denkbar und möglich. Bei dieser Ausgestaltung können grundsätzlich zwei Varianten zur rechnerischen Ermittlung des venösen Hämatokritwerts vorgesehen sein.

Bei einer ersten Variante werden der eingestellte Filtrationsvolumenstrom und ein Plasmawert $P_0$ des Bluts berücksichtigt. Um den venösen Hämatokritwert $H_v$ zu berechnen, wird zudem ein Gesamtvolumen des Bluts im Körper des Patienten benötigt. Dieses Gesamtvolumen kann über die Formel von Kaplan-Hakim bestimmt werden. Der venöse Hämatokritwert kann vor diesem Hintergrund wie folgt berechnet werden:

$$H_v = \frac{100 * (Q_p - (Plasma_0 - Q_F))}{Q_p}$$

Die hierfür benötigten Parameter liegen an dem zu überwachenden medizinischen Gerät üblicherweise bereits direkt oder jedenfalls indirekt vor.

Eine zweite Variante sieht vor, dass der Plasmawert des Bluts zeitaufgelöst betrachtet wird, indem die bekannten Größen des eingestellten Pumpvolumenstroms und des eingestellten Filtrationsvolumenstroms herangezogen werden. Der Filtrationsvolumenstrom kann naturgemäß lediglich aus dem Plasmaanteil des Bluts stammen, da das Hämatokrit selbst nicht die semipermeable Membran passieren kann. Ein Plasmastrom vor der Blutkammer kann aus dem eingestellten Pumpvolumenstrom und dem gemessenen arteriellen Hämatokritwert berechnet werden. Der (venöse) Plasmafluss nach der Blutbehandlungseinheit ist folglich die Differenz zwischen dem arteriellen Plasmafluss und dem Filtrationsvolumenstrom. Diese Zusammenhänge münden in:

$$H_v = 100 - \frac{(Q_p * (100 - H_a) - Q_F)}{Q_p - Q_F}$$

In weiterer Ausgestaltung des Verfahrens wird der Betrieb des Geräts bei mehreren zeitlich aufeinanderfolgenden extrakorporalen Blutbehandlungen des Patienten überwacht und jeweils ermittelte Kontrollparameter werden miteinander verglichen, wobei der Betrieb in Abhängigkeit des Vergleichs überwacht wird. Diese Ausgestaltung des Verfahrens ermöglicht es insbesondere, eine Veränderung der Gefäßzugänge und/oder der arteriovenösen Fistel zu erkennen. Verändert sich deren Zustand über der Zeit, d.h. über die einzelnen extrakorporalen Blutbehandlungen hinweg, kann dies anhand der Veränderung des Kontrollparameters über der Behandlungsanzahl erkannt werden. Dies ist eine besonders vorteilhafte Ausgestaltung der Erfindung. Der jeweils ermittelte Kontrollparameter kann in einer Datenbank abgespeichert werden. Zum Vergleichen der jeweils ermittelten Kontrollparameter können diese aus der besagten Datenbank abgerufen und entsprechend ausgewertet werden.

Das erfindungsgemäße Überwachungssystem ist für ein medizinisches Gerät zur extrakorporalen Blutbehandlung vorgesehen, wobei das Gerät eine Blutbehandlungseinheit mit einer Blutkammer, die zum Durchströmen mit Blut eingerichtet ist, und mit einer Behandlungskammer, die zum Durchströmen mit einer Behandlungsflüssigkeit eingerichtet ist und über eine semipermeable Membran von der Blutkammer getrennt ist, eine arterielle Fluidleitung, die einends mit einem Einlass der Blutkammer fluidleitend verbunden ist, und die andernends mit einem arteriellen Gefäßzugang eines Patienten fluidleitend verbindbar ist und die zum Durchströmen mit einem arteriellen Volumenstrom eingerichtet ist, eine venöse Fluidleitung, die einends mit einem Auslass der Blutkammer fluidleitend verbunden ist, die andernends mit einem venösen Gefäßzugang des Patienten fluidleitend verbindbar ist und die zum Durchströmen mit einem venösen Volumenstrom eingerichtet ist, und eine Blutpumpe, die zur Pumpförderung eines eingestellten Pumpvolumenstroms durch einen extrakorporalen Blutkreislauf eingerichtet ist, der die Blutkammer, die arterielle Fluidleitung und die venöse Fluidleitung umfasst, aufweist, wobei das Überwachungssystem aufweist: eine Ermittlungseinrichtung, die zum Ermitteln eines Kontrollparameters eingerichtet ist, der ein Verhältnis zwischen dem venösen Volumenstrom durch die venöse Fluidleitung und dem arteriellen Volumenstrom durch die arterielle Fluidleitung repräsentiert, und eine Überwachungseinrichtung, die zum Überwachen des Betriebs des medizinischen Geräts in Abhängigkeit des ermittelten Kontrollparameters eingerichtet ist. Die Ermittlungseinrichtung ist zum Ermitteln des Kontrollparameters gemäß der vorhergehenden Beschreibung des erfindungsgemäßen Verfahrens und/oder dessen Ausgestaltungen eingerichtet. Entsprechendes gilt, mutatis mutandis, für die Überwachungseinrichtung. Das Überwachungssystem ist bei einer Ausgestaltung ein von dem medizinischen Gerät gesondertes System. Bei einer bevorzugten Ausgestaltung ist das Überwachungssystem ein integraler Bestandteil des medizinischen Geräts. Mit anderen Worten ausgedrückt, wird das Überwachungssystem bei dieser bevorzugten Ausgestaltung von Komponenten des medizinischen Geräts gebildet.

In weiterer Ausgestaltung der Erfindung weist das Überwachungssystem eine Erfassungseinrichtung auf. Die Erfassungseinrichtung weist einen venösen Drucksensor, einen arteriellen Drucksensor, eine Blutdruckmessvorrichtung und wenigstens einen Hämatokritsensor auf. Dabei ist der venöse Drucksensor zum Erfassen eines venösen Fluiddrucks in der venösen Fluidleitung eingerichtet. Der arterielle Drucksensor ist zum Erfassen eines arteriellen Fluiddrucks in der arteriellen Fluidleitung eingerichtet. Die Blutdruckmessvorrichtung ist zum Erfassen eines mittleren arteriellen Drucks des Patienten eingerichtet. Der wenigstens eine Hämatokritsensor ist zum Erfassen eines arteriellen oder venösen Hämatokritwerts in der jeweiligen Fluidleitung eingerichtet. Weiter ist die Ermittlungseinrichtung bei dieser Ausgestaltung der Erfindung zum Ermitteln des Kontrollparameters als Funktion der mittels der Erfassungseinrichtung ermittelten Größen eingerichtet. Diese Ausgestaltung des Überwachungssystems ermöglicht eine approximierte Berechnung des Kontrollparameters auf Grundlage der Hagen-Poiseuille-Gleichung. Zur Vermeidung von Wiederholungen wird auf die diesbezügliche Offenbarung im Zusammenhang mit dem erfindungsgemäßen Verfahren verwiesen und ausdrücklich Bezug genommen.

In weiterer Ausgestaltung der Erfindung ist die Überwachungseinrichtung zur akustischen, optischen und/oder datenbasierten Ausgabe wenigstens eines Überwachungssignals eingerichtet. Das wenigstens eine Überwachungssignal kann bei einer akustischen und/oder optischen Ausgabe von medizinischem Personal wahrgenommen werden. Das medizinische Personal kann auf Grundlage des akustischen und/oder optischen Überwachungssignals Maßnahmen einleiten. Bei einer datenbasierten Ausgabe kann das wenigstens eine Überwachungssignal insbesondere einer Steuerung des medizinischen Geräts dienen. Mittels der Steuerung können Betriebsparameter des medizinischen Geräts verändert und/oder ein Betrieb des medizinischen Geräts beendet werden.

In weiterer Ausgestaltung der Erfindung ist das Überwachungssystem durch Komponenten des medizinischen Geräts gebildet. Dies ist eine bevorzugte Ausgestaltung der Erfindung.

Das erfindungsgemäße medizinische Gerät ist zur extrakorporalen Blutbehandlung eingerichtet und weist auf: eine Blutbehandlungseinheit mit einer Blutkammer, die zum Durchströmen mit Blut eingerichtet ist, und mit einer Behandlungskammer, die zum Durchströmen mit einer Behandlungsflüssigkeit eingerichtet ist und über eine semipermeable Membran von der Blutkammer getrennt ist, eine arterielle Fluidleitung, die einends mit einem Einlass der Blutkammer fluidleitend verbunden ist, die andernends mit einem arteriellen Gefäßzugang eines Patienten fluidleitend verbindbar ist und die zum Durchströmen mit einem arteriellen Volumenstrom eingerichtet ist, eine venöse Fluidleitung, die einends mit einem Auslass der Blutkammer fluidleitend verbunden ist, die andernends mit einem venösen Gefäßzugang des Patienten fluidleitend verbindbar ist und die zum Durchströmen mit einem venösen Volumenstrom eingerichtet ist, eine Blutpumpe, die zur Pumpförderung eines eingestellten Pumpvolumenstroms durch einen extrakorporalen Blutkreislauf eingerichtet ist, der die Blutkammer, die arterielle Fluidleitung und die venöse Fluidleitung umfasst, und ein Überwachungssystem gemäß der vorhergehenden Beschreibung. Das medizinische Gerät ist vorzugsweise ein Dialysegerät. Die Blutbehandlungseinheit kann in diesem Fall auch als Dialysator bezeichnet werden. Die Behandlungskammer ist in diesem Fall eine Dialysatkammer und die Behandlungsflüssigkeit ist ein Dialysat.

Weitere Vorteile und Merkmale der Erfindung ergeben sich aus der Beschreibung bevorzugter Ausführungsbeispiele der Erfindung, die anhand der Zeichnungen dargestellt sind.

Fig. 1     zeigt in schematisch vereinfachter Blockdarstellung eine Ausführungsform eines erfindungsgemäßen medizinischen Geräts mit einer Ausführungsform eines erfindungsgemäßen Überwachungssystems, das zum Ausführen einer Ausführungsform eines erfindungsgemäßen Verfahrens zum Überwachen des Betriebs des medizinischen Geräts eingerichtet ist,

Fig. 2     in schematisch vereinfachter sowie vergrößerter Detaildarstellung Endabschnitte zweier Fluidleitungen des medizinischen Geräts im Bereich patientenseitiger Gefäßzugänge,

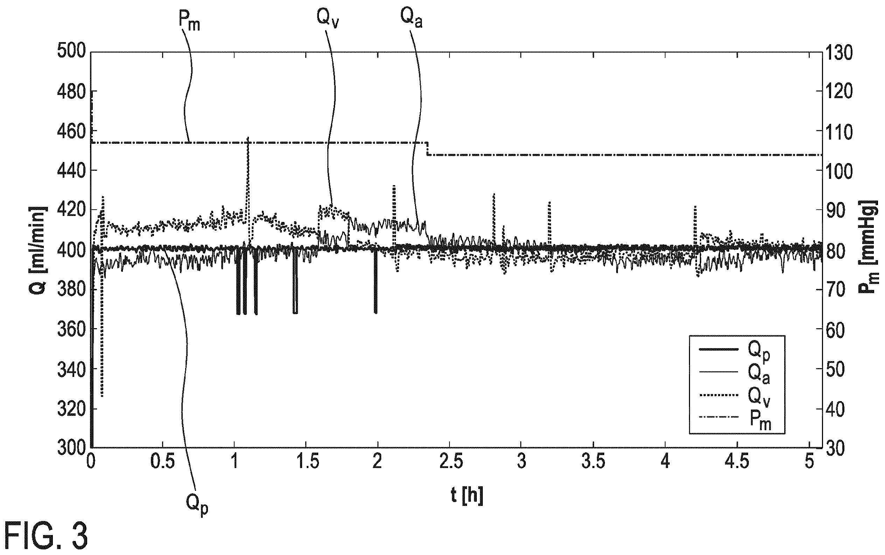

Fig. 3     ein Diagramm zur Verdeutlichung unterschiedlicher Volumenstromverläufe über der Zeitdauer einer extrakorporalen Blutbehandlung, sowie den Verlauf eines mittleren arteriellen Drucks des behandelten Patienten,

Fig. 4     ein Diagramm zur Verdeutlichung des zeitlichen Verlaufs eines Kontrollparameters und eines theoretischen Kontrollparameters über der Zeitdauer einer extrakorporalen Blutbehandlung,

Fig. 5     ein Diagramm zur Verdeutlichung eines auf empirischen Werten basierenden Zusammenhangs zwischen einer dynamischen Viskosität und einem Hämatokritwert und

Fig. 6     ein Diagramm zur Verdeutlichung der Änderung des Kontrollparameters über mehrere aufeinanderfolgende extrakorporale Blutbehandlungen hinweg.

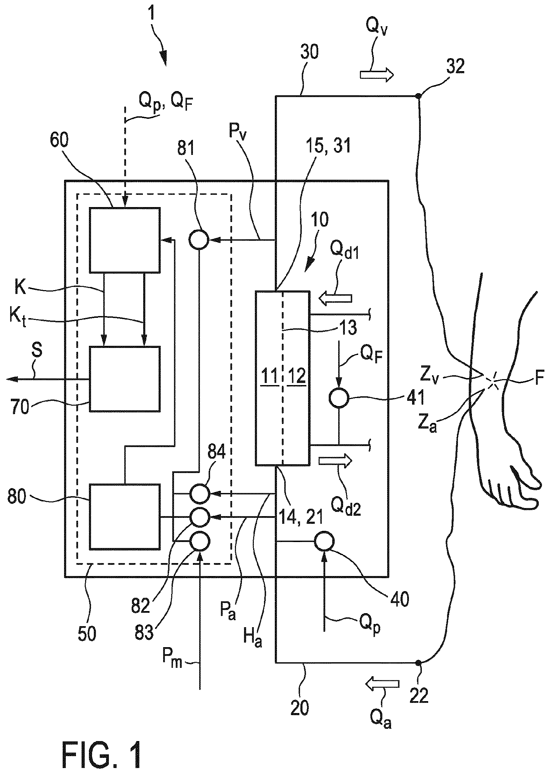

Gemäß Fig. 1 ist ein medizinisches Gerät 1 zur extrakorporalen Blutbehandlung vorgesehen und weist eine Blutbehandlungseinheit 10, eine arterielle Fluidleitung 20, eine venöse Fluidleitung 30 und eine Blutpumpe 40 auf.

Das medizinische Gerät 1 ist vorliegend in Form eines Dialysegeräts gestaltet. Die Behandlungseinheit 10 kann daher auch als Dialysator bezeichnet werden.

Die Blutbehandlungseinheit 10 weist eine Blutkammer 11 und eine Behandlungskammer 12 auf. Die Behandlungskammer 12 kann vorliegend auch als Dialysatkammer bezeichnet werden. Die Blutkammer 11 und die Behandlungskammer 12 sind mittels einer semipermeablen Membran 13 voneinander getrennt.

Die arterielle Fluidleitung 20 ist zwischen einem ersten Ende 21 und einem zweiten Ende 22 längserstreckt. Das erste Ende 21 der arteriellen Fluidleitung 20 ist fluidleitend mit einem Einlass 14 der Blutkammer 11 verbunden. Das zweite Ende 22 der arteriellen Fluidleitung 20 ist mit einem arteriellen Gefäßzugang $Z_a$ eines Patienten fluidleitend verbindbar.

Die venöse Fluidleitung 30 ist zwischen einem ersten Ende 31 und einem zweiten Ende 32 längserstreckt. Das erste Ende 31 der venösen Fluidleitung 30 ist fluidleitend mit einem Auslass 15 der Blutkammer 11 verbunden. Das zweite Ende 32 der venösen Fluidleitung 30 ist mit einem venösen Gefäßzugang $Z_v$ des Patienten fluidleitend verbindbar.

Die Blutkammer 11 liegt stromaufwärts der arteriellen Fluidleitung 20. Die venöse Fluidleitung 30 liegt stromaufwärts des Blutkammer 11.

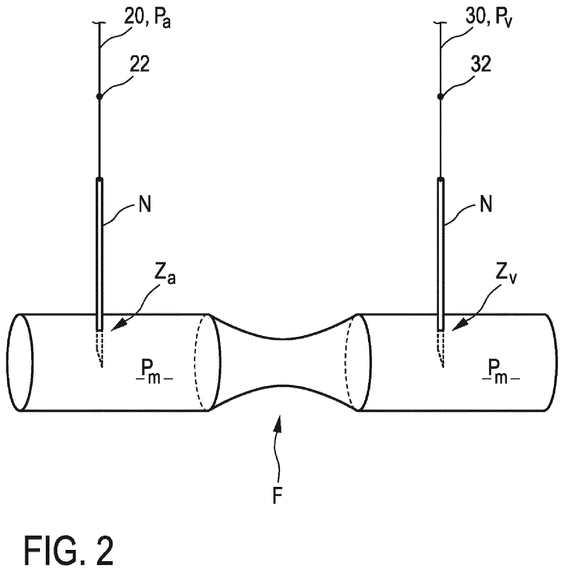

Im Betrieb des medizinischen Geräts 1 sind die beiden besagten zweiten Enden 22, 32 der Fluidleitungen 20, 30 mittels jeweils einer Nadel N und gegebenenfalls weiterer Schlauchabschnitte (ohne Bezugszeichen) mit dem jeweiligen Gefäßzugang $Z_a$, $Z_v$ fluidleitend verbunden (siehe Fig. 2). Die Nadeln N können auch als Kanüle oder Hohlnadel bezeichnet werden.

Wie weiter anhand Fig. 2 verdeutlicht ist, sind die Gefäßzugänge $Z_a$, $Z_v$ vorliegend an einer arteriovenösen Fistel F des Patienten angeordnet. Die arteriovenöse Fistel ist in der exemplarisch gezeigten Situation an einem Unterarm des Patienten angeordnet (siehe Fig. 1) und wird auf eine dem Fachmann bekannte Weise chirurgisch hergestellt. Die arteriovenöse Fistel F stellt eine Art fluidischen Kurzschluss zwischen einer Vene und einer Arterie des Patienten her.

Während der extrakorporalen Blutbehandlung gelangt das zu behandelnde Blut des Patienten über den arteriellen Gefäßzugang $Z_a$ in die arterielle Fluidleitung 20 und von deren erstem Ende 21 über den Einlass 14 in die Blutkammer 11. Von dort gelangt das Blut über den Auslass 15 in die venöse Fluidleitung 30 und über deren zweites Ende 32 und den venösen Gefäßzugang $Z_v$ zurück in den Blutkreislauf des Patienten.

Die arterielle Fluidleitung 20, die Blutkammer 11 und die venöse Fluidleitung 30 bilden einen/den extrakorporalen Blutkreislauf des medizinischen Geräts 1. Die Blutpumpe 40 dient zur Pumpförderung des Bluts innerhalb des extrakorporalen Blutkreislaufs. Die Pumpförderung, genauer: ein Pumpvolumenstrom $P_Q$ der Blutpumpe 40, ist einstellbar.

Die Behandlungskammer 12 wird im Betrieb des medizinischen Geräts 1 von einer Behandlungsflüssigkeit durchströmt, die vorliegend als Dialysat bezeichnet werden kann. Die Durchströmung erfolgt im Gegenstrom zu dem Blutstrom in der Blutkammer 11.

Beim Durchströmen der Blutkammer 11 wird harnpflichtige Flüssigkeit aus dem Blutstrom über die semipermeable Membran 13 in die Behandlungskammer 12 übergeleitet und mit der dortigen Flüssigkeitsströmung abtransportiert.

Die Menge der dem Blutstrom zu entziehenden Flüssigkeit pro Zeiteinheit kann an dem medizinischen Gerät 1 eingestellt werden und wird als Filtrationsvolumenstrom $Q_F$ bezeichnet.

Die Einstellung des Filtrationsvolumenstroms $Q_F$ erfolgt vorliegend über eine Filtrationspumpe 41. Die Filtrationspumpe 41 fördert die Behandlungsflüssigkeit durch die Behandlungskammer 12. Dabei liegt an einem Einlass (ohne Bezugszeichen) der Behandlungskammer 12 ein Volumenstrom $Q_{d1}$ an. An einem Auslass (ohne Bezugszeichen) der Behandlungskammer 12 liegt ein Volumenstrom $Q_{d2}$ an. Der an dem Auslass anliegende Volumenstrom $Q_{d2}$ ergibt sich als Summe aus dem am Einlass eingeleiteten Volumenstrom $Q_{d1}$ und dem Filtrationsvolumenstrom $Q_F$.

Infolge der Ableitung des Filtrationsvolumenstroms $Q_F$ aus dem extrakorporalen Blutkreislauf ergibt sich folglich auch eine (rechnerische) Differenz zwischen den Volumenströmen innerhalb der Fluidleitungen 20, 30, wobei gilt

$$Q_v = Q_a - Q_F$$

Dabei gibt $Q_a$ einen arteriellen Volumenstrom in der arteriellen Fluidleitung 20 an. $Q_v$ gibt einen venösen Volumenstrom in der venösen Fluidleitung 30 an.

Das medizinische Gerät 1 weist zudem ein Überwachungssystem 50 auf. Das Überwachungssystem 50 dient einer Überwachung des Betriebs des medizinischen Geräts 1. Im Speziellen kann mittels des Überwachungssystems 50 überwacht werden, ob eine Beeinträchtigung des Blutflusses innerhalb des extrakorporalen Blutkreislaufs vorliegt. Zudem kann überwacht werden, ob die Verbindung zwischen dem extrakorporalen Blutkreislauf und dem Blutkreislauf des Patienten über die Gefäßzugänge $Z_a$, $Z_v$ vorliegt. Überdies können Veränderungen der arteriovenösen Fistel F erkannt werden.

Das Überwachungssystem 50 ist bei der gezeigten Ausführungsform ein integraler Bestandteil des medizinischen Geräts 1. Mit anderen Worten wird das Überwachungssystem 50 durch Komponenten des medizinischen Geräts 1 gebildet. Bei einer in den Figuren nicht gezeigten Ausführungsform ist das Überwachungssystem nicht in das medizinische Gerät integriert.

Das Überwachungssystem 50 weist eine Ermittlungseinrichtung 60 und eine Überwachungseinrichtung 70 auf.

Die Ermittlungseinrichtung 60 ist zum Ermitteln eines Kontrollparameters K eingerichtet. Der Kontrollparameter K repräsentiert ein Verhältnis zwischen dem venösen Volumenstrom $Q_v$ und dem arteriellen Volumenstrom $Q_a$, wobei

$$K = \frac{Q_v}{Q_a}$$

Die Überwachungseinrichtung 70 ist zum Überwachen des Betriebs des medizinischen Geräts 1 in Abhängigkeit des ermittelten Kontrollparameters K eingerichtet.

Bei der gezeigten Ausführungsform ist die Überwachungseinrichtung 70 zur Ausgabe eines Überwachungssignals S eingerichtet. Das Überwachungssignal S kann akustisch, optisch und/oder datenbasiert ausgegeben werden.

Anhand des optisch und/oder akustisch ausgegebenen Kontrollsignals S kann medizinisches Personal erkennen, ob eine Beeinträchtigung oder Störung vorliegt, so dass Betriebsparameter des medizinischen Geräts 1 manuell verändert werden können.

Eine datenbasierte Ausgabe des Kontrollsignals S kann einer automatischen Steuerung des medizinischen Geräts 1 zugrunde gelegt werden. Beispielsweise kann die Blutpumpe 40 und/oder die Filtrationspumpe 41 in Abhängigkeit des Kontrollsignals S angesteuert werden.

Die Ermittlungseinrichtung 60 ist bei unterschiedlichen Ausführungsformen auf unterschiedliche Weise zur Ermittlung des Kontrollparameters K eingerichtet. Beispielsweise kann der Kontrollparameter K auf Grundlage einer direkten messtechnischen Erfassung des arteriellen Volumenstroms $Q_a$ und des venösen Volumenstroms $Q_v$ ermittelt werden. Vorliegend wird der Kontrollparameter K stattdessen approximiert ermittelt/berechnet. Diese approximierte Ermittlung/Berechnung erfolgt auf der Grundlage von an dem medizinischen Gerät 1 eingestellten Betriebsparametern und/oder erfassten Messgrößen, die in einem mittelbaren approximierten physikalischen Zusammenhang mit den Volumenströmen $Q_a$ und $Q_v$ stehen.

Bei der gezeigten Ausführungsform weist das Überwachungssystem 50 zur messtechnischen Erfassung der besagten Messgrößen eine Erfassungseinrichtung 80 auf. Die Erfassungseinrichtung 80 weist einen venösen Drucksensor 81, einen arteriellen Drucksensor 82, eine Blutdruckmessvorrichtung 83 und einen Hämatokritsensor 84 auf.

Der venöse Drucksensor 81 ist zum Erfassen eines venösen Fluiddrucks $P_v$ in der venösen Fluidleitung 30 eingerichtet. Der arterielle Drucksensor 82 ist zum Erfassen eines arteriellen Fluiddrucks $P_a$ in der arteriellen Fluidleitung 20 eingerichtet. Die Blutdruckmessvorrichtung ist zum Erfassen eines mittleren arteriellen Drucks $P_m$ eingerichtet. Der Hämatokritsensor 84 ist zum Erfassen eines arteriellen Hämatokritwerts $H_a$ des Blutstroms in der arteriellen Fluidleitung 20 eingerichtet.

Die Ermittlung des Kontrollparameters K erfolgt vorliegend unter Verwendung der mittels der Erfassungseinrichtung 80 erfassten Messgrößen $P_v$, $P_a$, $P_m$,

$H_a$ und auf Grundlage der Hagen-Poiseuille-Gleichung. Die Hagen-Poiseuille-Gleichung, die oftmals auch als Gesetz von Hagen-Poiseuille bezeichnet wird, beschreibt den Volumenstrom bei einer laminaren stationären Strömung eines Newton'schen Fluids in einem Rohr mit einem Radius r und einer Länge l. Folglich gilt approximiert für die beiden Volumenströme in den Fluidleitungen 20, 30:

$$Q_a = \frac{\pi * r_a^4 * (\Delta P_a)}{8 * \eta_a * l_a}$$

$$Q_v = \frac{\pi * r_v^4 * (\Delta P_v)}{8 * \eta_v * l_v}$$

Für den Kontrollparameter K ergibt sich folglich

$$K = Q_v/Q_a = \frac{\eta_a * \Delta P_v}{\eta_v * \Delta P_a}$$

wobei durch die Bildung des Verhältnisses/Quotienten die Länge und der Radius aus der Bestimmungsgleichung gekürzt werden können. Diese beiden Größen unterliegen herstellungsbedingten Toleranzen. Da der Radius r zudem exponentiell in die Bestimmungsgleichung eingeht, haben Abweichungen einen großen Einfluss auf das Ergebnis. Die Ermittlung des Kontrollparameters als Quotient aus den beiden Volumenströmen $Q_a$, $Q_v$ vermeidet hiermit einhergehende Ungenauigkeiten.

In Anbetracht obiger Gleichung lässt sich feststellen, dass für die approximierte Ermittlung des Volumenstromverhältnisses, d.h. des Kontrollparameters K, Differenzdrücke, genauer ein venöser Differenzdruck $P_v$-$P_m$ und ein arterieller Differenzdruck $P_m$-$P_a$, ermittelt werden. Die besagten Druckwerte werden mittels des venösen Drucksensors 81, des arteriellen Drucksensors 82 und der Blutdruckmessvorrichtung 83 ermittelt. Die Ermittlung des mittleren arteriellen Drucks $P_m$ erfolgt nicht-invasiv und auf eine dem Fachmann grundsätzlich bekannte Weise.

Weiter werden eine arterielle Viskosität $\eta_a$ und eine venöse Viskosität $\eta_v$ ermittelt. Die Ermittlung der beiden Viskositäten erfolgt näherungsweise. Alternativ ist grundsätzlich auch eine direkte messtechnische Erfassung der Viskositäten denkbar und möglich.

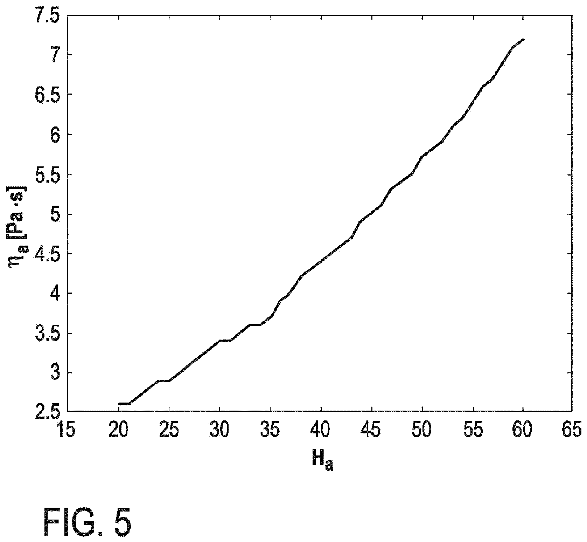

Bei der gezeigten Ausführungsform wird die arterielle Viskosität $\eta_a$ als Funktion des gemessenen arteriellen Hämatokritwerts $H_a$ ermittelt. Die Ermittlungseinrichtung 60 ist entsprechend eingerichtet. Die Ermittlung erfolgt auf Grundlage des in Fig. 5 exemplarisch verdeutlichten Zusammenhangs zwischen den besagten Größen. Der dargestellte Zusammenhang basiert auf empirischen Werten und lässt eine Ermittlung der arteriellen Viskosität $\eta_a$ in Abhängigkeit des gemessenen arteriellen Hämatokritwerts $H_a$ zu. Die Ermittlung der arteriellen Viskosität $\eta_a$ kann beispielsweise auf Grundlage einer linearen Funktion erfolgen, die den dargestellten empirischen Zusammenhang näherungsweise wiedergibt. Alternativ oder zusätzlich können die besagten empirischen Werte in einer hierfür eingerichteten Speichereinheit des medizinischen Geräts 1, die insbesondere der Ermittlungseinrichtung 60 zugeordnet sein kann, abgespeichert und abrufbar sein.

Die venöse Viskosität $\eta_v$ wird vorliegend approximiert ermittelt. Dabei sind unterschiedliche Approximationen denkbar und möglich:
Bei einer Variante wird die venöse Viskosität $\eta_v$ als Funktion des an dem medizinischen Gerät 1 eingestellten Pumpvolumenstroms $Q_p$, des eingestellten Filtrationsvolumenstroms $Q_F$ und des gemessenen arteriellen Hämatokritwerts $H_a$ ermittelt. Die aus dieser Variante hervorgehende Bestimmungsgleichung folgt als

$$H_v = 100 - \frac{(Q_p * (100 - H_a) - Q_F)}{Q_p - Q_F}$$

Gemäß einer weiteren Variante wird zunächst der venöse Hämatokritwert $H_v$ nach der Blutkammer 11 und damit in der venösen Fluidleitung 30 rechnerisch ermittelt. Diese rechnerische Ermittlung erfolgt auf Grundlage von an dem medizinischen Gerät eingestellten Betriebsparametern, nämlich dem Pumpvolumenstrom $Q_p$ und dem Filtrationsvolumenstrom $Q_F$, sowie dem Plasmawert $Plasma_0$ des Bluts. Der Plasmawert $Plasma_0$ liegt an dem medizinischen Gerät 1 üblicherweise als Messwert vor und kann beispielsweise mittels der Erfassungseinrichtung 80 und einem hierfür geeigneten Sensor oder dergleichen erfasst werden. Die für die rechnerische Ermittlung des venösen Hämatokritwerts $H_v$ vorliegend maßgebliche Gleichung folgt als:

$$H_v = \frac{100 * (Q_p - (Plasma_0 - Q_F))}{Q_p}$$

Ausgehend von dem auf diese Weise rechnerisch ermittelten venösen Hämatokritwert $H_v$ kann die venöse Viskosität $\eta_v$ auf Grundlage des in Fig. 5 wiedergegebenen empirischen Zusammenhangs und somit analog zu der arteriellen Viskosität $\eta_a$ ermittelt werden.

Der auf diese Weise ermittelte Kontrollparameter K stellt einen Vergleich zwischen den approximierten Volumenströmen $Q_a$, $Q_v$ dar. Eine Überwachung des Kontrollparameters K lässt folglich einen Rückschluss auf übermäßige Abweichungen zwischen den Volumenströmen $Q_a$, $Q_v$ zu. Ursächlich für solche Abweichungen können Beeinträchtigungen oder Veränderungen an den Fluidleitungen 20, 30, den Gefäßzugängen $Z_a$, $Z_v$ und/oder der arteriovenösen Fistel F sein.

Zwecks Überwachung wird bei einer Ausführungsform lediglich der Kontrollparameter K ermittelt und der Überwachung, d.h. der Ausgabe des Überwachungssignals S, zugrunde gelegt.

Bei der gezeigten Ausführungsform wird zudem die Änderung des Kontrollparameters K über der Zeit erfasst. Die Überwachung erfolgt in Abhängigkeit der erfassten zeitlichen Änderung des Kontrollparameters K.

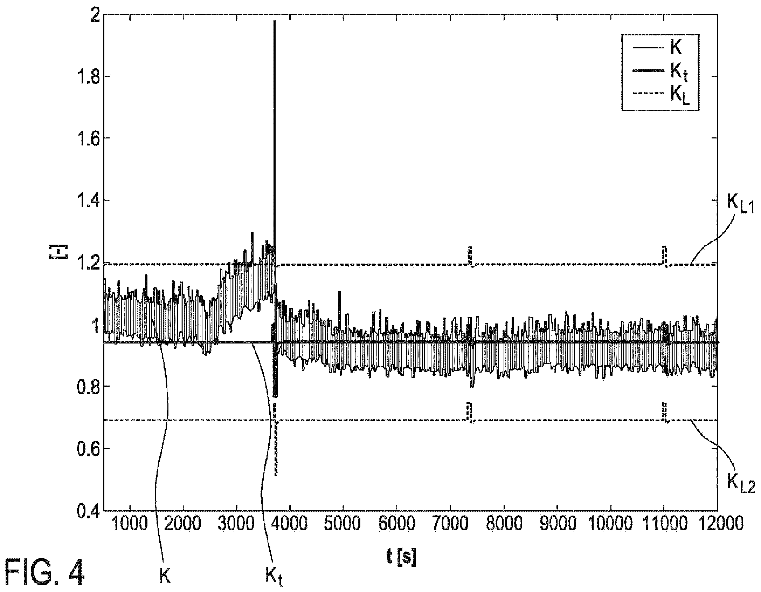

Fig. 4 zeigt einen exemplarischen Verlauf des Kontrollparameters K über der Zeit t, die sich in dem gezeigten Beispiel über die Dauer einer extrakorporalen Blutbehandlung erstreckt. In dem Beispiel ist ein Anstieg des Kontrollparameters K in dem Zeitraum zwischen etwa 2.000 s und 4.000 s gezeigt. Dieser Anstieg kann unterschiedliche Ursachen haben:

Beispielsweise können die venöse Fluidleitung 30, die an der venösen Fluidleitung 30 angeschlossene Nadel N und/oder der venöse Gefäßzugang $Z_v$ beeinträchtigt sein. Hierdurch steigt der venöse Differenzdruck, der Kontrollparameter K steigt über der Zeit t an.

Ein Sinken des Kontrollparameters K über der Zeit t kann insbesondere folgende Ursachen haben:

Durch eine Beeinträchtigung der arteriellen Fluidleitung 20, der endseitig angeschlossenen Nadel N und/oder des arteriellen Gefäßzugangs $Z_a$ ergibt sich ein größerer arterieller Differenzdruck, der Kontrollparameter K sinkt.

Ein Lösen der venösen Fluidleitung 30 von dem venösen Gefäßzugang $Z_v$, beispielsweise durch eine Diskonnektion der betreffenden Nadel N, führt dazu, dass der gemessene venöse Fluiddruck $P_v$ sinkt. Folglich sinkt auch der Kontrollparameter K. Entsprechendes gilt sinngemäß für ein Lösen der arteriellen Fluidleitung 20 und/oder eine Diskonnektion der betreffenden Nadel N. Hierdurch steigt der arterielle Fluiddruck $P_a$, wodurch folglich der Kontrollparameter K sinkt.

Im Unterschied zu aus dem Stand der Technik bekannten Überwachungsverfahren, die lediglich eine Überwachung anhand der gemessenen arteriellen und venösen Fluiddrücke vorsehen, erlaubt das vorliegende Überwachungsverfahren/Überwachungssystem zusätzlich eine Berücksichtigung etwaiger Viskositätsänderungen des Bluts sowie Änderungen des Blutdrucks des Patienten. Dies erlaubt eine zuverlässigere und somit für den Patienten sicherere Überwachung.

Überdies kann eine Veränderung der arteriovenösen Fistel F erkannt werden. Eine Veränderung der in Fig. 2 schematisch angedeuteten Engstelle zwischen dem arteriellen Gefäßzugang $Z_a$ und dem venösen Gefäßzugang $Z_v$ geht mit einer Veränderung der dort vorherrschenden Blutdrücke einher. Aufgrund der besagten Änderung der Drücke ändert sich folglich auch der Kontrollparameter K.

Bei der gezeigten Ausführungsform erfolgt zudem ein Abgleich des ermittelten Kontrollparameters K mit einem Vergleichsparameter, der vorliegend als theoretischer Kontrollparameter $K_t$ bezeichnet wird. Der theoretische Kontrollparameter repräsentiert ein theoretisches Volumenstromverhältnis und wird als Funktion des eingestellten Pumpvolumenstroms $Q_p$ und des eingestellten Filtrationsvolumenstroms $Q_F$ bestimmt. Dabei gilt

$$K_t = \frac{Q_p - Q_F}{Q_p}$$

Durch einen Vergleich des Kontrollparameters K mit dem theoretischen Kontrollparameter $K_t$ kann der Einfluss einer Änderung des eingestellten Filtrationsvolumenstroms $Q_F$ berücksichtigt werden.

Der Verlauf des theoretischen Kontrollparameters $K_t$ über der Zeit t ist exemplarisch in dem Diagramm nach Fig. 4 eingezeichnet. Auf Grundlage des theoretischen Kontrollparameters $K_t$ lassen sich ein oberer Grenzwert $K_{L1}$ und ein unterer Grenzwert $K_{L2}$ für den Kontrollparameter K ermitteln. Überschreitet der Kontrollparameter K den oberen Grenzwert $K_{L1}$ oder unterschreitet der Kontrollparameter K den unteren Grenzwert $K_{L2}$, lässt dies auf eine Beeinträchtigung des Betriebs des medizinischen Geräts 1 schließen. Zudem gibt eine zu starke Abweichung zwischen dem Kontrollparameter K und dem theoretischen Kontrollparameter $K_t$ einen Hinweis auf eine etwaige Veränderung der arteriovenösen Fistel F.

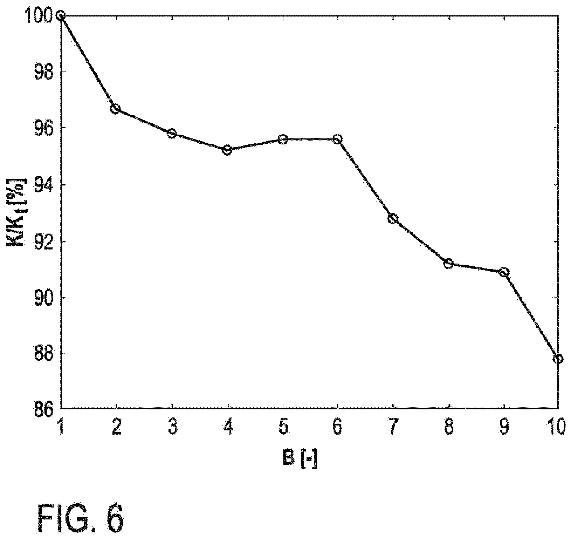

Das vorliegende Überwachungsverfahren sieht zudem vor, dass der Betrieb des medizinischen Geräts 1 bei mehreren zeitlich aufeinanderfolgenden extrakorporalen Blutbehandlungen des Patienten überwacht wird. Die hierbei ermittelten Kontrollparameter werden miteinander verglichen. Eine solche Überwachung ist anhand Fig. 6 verdeutlicht. Dort ist das Verhältnis zwischen dem Kontrollparameter K und dem theoretischen Kontrollparameter $K_t$ über einer Anzahl B an extrakorporalen Blutbehandlungen ein- und desselben Patienten aufgetragen. Die Veränderung des besagten Verhältnisses erlaubt einen Rückschluss auf eine zeitliche Veränderung der arteriovenösen Fistel F. Insbesondere kann der anhand Fig. 6 verdeutlichte Verlauf des Verhältnisses K/Kt auf eine übermäßige Verengung oder einen Verschluss der arteriovenösen Fistel F zwischen den beiden Gefäßzugängen $Z_a$, $Z_v$ hindeuten.

**Patentansprüche**

1. Verfahren zum Überwachen des Betriebs eines medizinischen Geräts (1) zur extrakorporalen Blutbehandlung, das Gerät (1) aufweisend

    eine Blutbehandlungseinheit (10) mit einer Blutkammer (11), die von Blut durchströmt wird, und mit einer Behandlungskammer (12), die von einer Blutbehandlungsflüssigkeit durchströmt wird und über eine semipermeable Membran (13) von der Blutkammer (11) getrennt ist,
    eine arterielle Fluidleitung (20), die einends mit einem Einlass (14) der Blutkammer (11) und andernends mit einem arteriellen Gefäßzugang ($Z_a$) eines Patienten fluidleitend verbunden ist und von einem arteriellen Volumenstrom ($Q_a$)

durchströmt wird,
eine venöse Fluidleitung (30), die einends mit einem Auslass (15) der Blutkammer (11) und andernends mit einem venösen Gefäßzugang ($Z_v$) des Patienten fluidleitend verbunden ist und von einem venösen Volumenstrom ($Q_v$) durchströmt wird, und
eine Blutpumpe (40), die zum Fördern eines Pumpvolumenstroms ($Q_p$) durch einen extrakorporalen Blutkreislauf, welcher die Blutkammer (11), die arterielle Fluidleitung (20) und die venöse Fluidleitung (30) umfasst, eingerichtet ist, wobei das Verfahren die Schritte aufweist:

Ermitteln eines Kontrollparameters (K), der ein Verhältnis zwischen dem venösen Volumenstrom ($Q_v$) durch die venöse Fluidleitung (30) und dem arteriellen Volumenstrom ($Q_a$) durch die arterielle Fluidleitung (20) repräsentiert;
Ermitteln eines theoretischen Kontrollparameters ($K_t$), der ein theoretisches Verhältnis zwischen dem venösen Volumenstrom ($Q_v$) und dem arteriellen Volumenstrom ($Q_a$) repräsentiert, wobei der theoretische Kontrollparameter ($K_t$) als Funktion des Pumpvolumenstroms ($Q_p$) und eines Filtrationsvolumenstroms ($Q_F$), der über die semipermeable Membran (13) aus der Blutkammer (11) abgeleitet wird, ermittelt wird;
Vergleichen des ermittelten Kontrollparameters (K) mit dem ermittelten theoretischen Kontrollparameter ($K_t$);
Überwachen des Betriebs in Abhängigkeit des Vergleichs.

2. Verfahren nach Anspruch 1, umfassend die Schritte:

Erfassen einer Änderung des Kontrollparameters (K) über der Zeit (t);
Überwachen des Betriebs in Abhängigkeit der erfassten zeitlichen Änderung des Kontrollparameters (K).

3. Verfahren nach einem der vorhergehenden Ansprüche, wobei der arterielle Volumenstrom ($Q_a$) und der venöse Volumenstrom ($Q_v$) jeweils unmittelbar messtechnisch erfasst werden.

4. Verfahren nach Anspruch 1 oder 2, wobei der arterielle Volumenstrom ($Q_a$) und der venöse Volumenstrom ($Q_v$) auf Grundlage der Hagen-Poiseuille-Gleichung approximiert ermittelt werden.

5. Verfahren nach Anspruch 4, wobei das approximierte Ermitteln umfasst:

Ermitteln eines venösen Differenzdrucks ($P_v$-$P_m$) zwischen der venösen Fluidleitung (30) und dem venösen Gefäßzugang ($Z_v$) und eines arteriellen Differenzdrucks ($P_m$-$P_a$) zwischen der arteriellen Fluidleitung (20) und dem arteriellen Gefäßzugang ($Z_a$);
Ermitteln einer arteriellen Viskosität ($\eta_a$) des arteriellen Volumenstroms ($Q_a$) und einer venösen Viskosität ($\eta_v$) des venösen Volumenstroms ($Q_v$);
Berechnen des Kontrollparameters (K) als Funktion der erfassten Differenzdrücke ($P_v$-$P_m$, $P_m$-$P_a$) und der erfassten Viskositäten ($\eta_a$, $\eta_v$).

6. Verfahren nach Anspruch 5, wobei das Ermitteln der Differenzdrücke ($P_v$-$P_m$, $P_m$-$P_a$) umfasst:

Messen eines venösen Fluiddrucks ($P_v$) in der venösen Fluidleitung (30) und eines arteriellen Fluiddrucks ($P_a$) in der arteriellen Fluidleitung (20);
Erfassen, insbesondere nicht-invasives Messen, eines mittleren arteriellen Drucks ($P_m$) des Patienten.

7. Verfahren nach Anspruch 5 oder 6, wobei das Ermitteln der Viskositäten ($\eta_a$, $\eta_v$) umfasst:

Messen eines arteriellen Hämatokritwerts ($H_a$) des Bluts in der arteriellen Fluidleitung (20) und/oder eines venösen Hämatokritwerts ($H_v$) des Bluts in der venösen Fluidleitung (30);
Ermitteln der arteriellen Viskosität ($\eta_v$) als Funktion des gemessenen arteriellen Hämatokritwerts ($H_a$) und/oder der venösen Viskosität ($\eta_v$) als Funktion des gemessenen venösen Hämatokritwerts ($H_v$).

8. Verfahren nach Anspruch 7, wobei der arterielle Hämatokritwert ($H_a$) messtechnisch erfasst wird und die venöse Viskosität ($\eta_v$) approximiert ermittelt wird als Funktion des Pumpvolumenstroms ($Q_p$), des Filtrationsvolumenstroms ($Q_F$) und des messtechnisch erfassten arteriellen Hämatokritwerts ($H_a$).

9. Verfahren nach einem der vorhergehenden Ansprüche, wobei der Betrieb des Geräts (1) bei mehreren zeitlich aufeinanderfolgenden Behandlungen des Patienten überwacht und hierbei jeweils ermittelte Kontrollparameter miteinander verglichen werden, wobei der Betrieb in Abhängigkeit des Vergleichs überwacht wird.

10. Überwachungssystem (50) für ein medizinisches Gerät (1) zur extrakorporalen Blutbehandlung, das Gerät (1) aufweisend

eine Blutbehandlungseinheit (10) mit einer Blutkammer (11), die zum Durchströmen mit Blut eingerichtet ist, und mit einer Behandlungskammer (12), die zum Durchströmen mit einer Behandlungsflüssigkeit eingerichtet und über eine semipermeable Membran (13) von der Blutkammer (11) getrennt ist,
eine arterielle Fluidleitung (20), die einends mit einem Einlass (14) der Blutkammer (11) fluidleitend verbunden ist, die andernends mit einem arteriellen Gefäßzugang ($Z_a$) eines Patienten fluidleitend verbindbar ist und die zum Durchströmen mit einem arteriellen Volumenstrom ($Q_a$) eingerichtet ist,
eine venöse Fluidleitung (30), die einends mit einem Auslass (15) der Blutkammer (11) fluidleitend verbunden ist, die andernends mit einem venösen Gefäßzugang ($Z_v$) des Patienten fluidleitend verbindbar ist und die zum Durchströmen mit einem venösen Volumenstrom ($Q_v$) eingerichtet ist, und
eine Blutpumpe (40), die zur Pumpförderung eines Pumpvolumenstroms ($Q_p$) durch einen extrakorporalen Blutkreislauf eingerichtet ist, der die Blutkammer (11), die arterielle Fluidleitung (20) und die venöse Fluidleitung (30) umfasst,
wobei das Überwachungssystem (50) aufweist:

eine Ermittlungseinrichtung (60), die eingerichtet ist zum

Ermitteln eines Kontrollparameters (K), der ein Verhältnis zwischen dem venösen Volumenstrom ($Q_v$) durch die venöse Fluidleitung (30) und dem arteriellen Volumenstrom ($Q_a$) durch die arterielle Fluidleitung (20) repräsentiert;
Ermitteln eines theoretischen Kontrollparameters ($K_t$), der ein theoretisches Verhältnis zwischen dem venösen Volumenstrom ($Q_v$) und dem arteriellen Volumenstrom ($Q_a$) repräsentiert, wobei die Ermittlungseinrichtung (60) dazu eingerichtet ist, den theoretischen Kontrollparameter ($K_t$) als Funktion des Pumpvolumenstroms ($Q_p$) und eines Filtrationsvolumenstroms ($Q_F$), der über die semipermeable Membran (13) aus der Blutkammer (11) abgeleitet wird, zu ermitteln;
Vergleichen des ermittelten Kontrollparameters (K) mit dem ermittelten theoretischen Kontrollparameter ($K_t$);

eine Überwachungseinrichtung (70), die zum Überwachen des Betriebs des medizinischen Geräts (1) in Abhängigkeit des Vergleichs zwischen dem ermittelten Kontrollparameter (K) und dem ermittelten theoretischen Kontrollparameter ($K_t$) eingerichtet ist.

11. Überwachungssystem (50) nach Anspruch 10, **dadurch gekennzeichnet, dass** eine Erfassungseinrichtung (80) vorhanden ist und einen venösen Drucksensor (81), der zum Erfassen eines venösen Fluiddrucks ($P_v$) in der venösen Fluidleitung (30) eingerichtet ist, einen arteriellen Drucksensor (82), der zum Erfassen eines arteriellen Fluiddrucks ($P_a$) in der arteriellen Fluidleitung (20) eingerichtet ist, eine Blutdruckmessvorrichtung (83), die zum Erfassen eines mittleren arteriellen Drucks ($P_m$) des Patienten eingerichtet ist, und wenigstens einen Hämatokritsensor (84) aufweist, der zum Erfassen eines arteriellen oder venösen Hämatokritwerts ($H_a$, $H_v$) in der jeweiligen Fluidleitung (20, 30) eingerichtet ist, wobei die Ermittlungseinrichtung (60) zum Ermitteln des Kontrollparameters (K) als Funktion der mittels der Erfassungseinrichtung (80) erfassten Größen eingerichtet ist.

12. Überwachungssystem (50) nach Anspruch 10 oder 11, **dadurch gekennzeichnet, dass** die Überwachungseinrichtung (70) zur akustischen, optischen und/oder datenbasierten Ausgabe wenigstens eines Überwachungssignals (S) eingerichtet ist.

13. Überwachungssystem (50) nach einem der Ansprüche 10 bis 12, wobei das Überwachungssystem (50) durch Komponenten des medizinischen Geräts (1) gebildet ist.

14. Medizinisches Gerät (1) zur extrakorporalen Blutbehandlung aufweisend

eine Blutbehandlungseinheit (10) mit einer Blutkammer (11), die zum Durchströmen mit Blut eingerichtet ist, und mit einer Behandlungskammer (12), die zum Durchströmen mit einer Behandlungsflüssigkeit eingerichtet und über eine semipermeable Membran (13) von der Blutkammer (11) getrennt ist,
eine arterielle Fluidleitung (20), die einends mit einem Einlass (14) der Blutkammer (11) fluidleitend verbunden ist, die andernends mit einem arteriellen Gefäßzugang ($Z_a$) eines Patienten fluidleitend verbindbar ist und die zum Durchströmen mit einem arteriellen Volumenstrom ($Q_a$) eingerichtet ist,
eine venöse Fluidleitung (30), die einends mit einem Auslass (15) der Blutkammer (11) fluidleitend verbunden ist, die andernends mit einem venösen Gefäßzugang ($Z_v$) des Patienten fluidleitend verbindbar ist und die zum Durchströmen mit einem venösen Volumenstrom ($Q_v$) eingerichtet ist, und

eine Blutpumpe (40), die zur Pumpförderung eines Pumpvolumenstroms ($Q_p$) durch einen extrakorporalen Blutkreislauf eingerichtet ist, der die Blutkammer (11), die arterielle Fluidleitung (20) und die venöse Fluidleitung (30) umfasst,
und aufweisend ein Überwachungssystem (50) nach einem der Ansprüche 10 bis 13.

## Claims

1. Method for monitoring the operation of a medical device (1) for extracorporeal blood treatment, the device (1) comprising

   a blood treatment unit (10) having a blood chamber (11) that is traversed by blood and a treatment chamber (12) that is traversed by a blood treatment fluid and separated from the blood chamber (11) by way of a semipermeable membrane (13),
   an arterial fluid line (20) that is connected in fluid-conveying fashion at one end to an inlet (14) in the blood chamber (11) and at the other end to an arterial vessel access ($Z_a$) of a patient and that is traversed by an arterial volume flow ($Q_a$),
   a venous fluid line (30) that is connected in fluid-conveying fashion at one end to an outlet (15) from the blood chamber (11) and at the other end to a venous vessel access ($Z_v$) of the patient and that is traversed by a venous volume flow ($Q_v$), and
   a blood pump (40) that is configured to convey a pump volume flow ($Q_p$) through an extracorporeal blood circuit comprising the blood chamber (11), the arterial fluid line (20) and the venous fluid line (30),
   wherein the method includes the steps of:

   ascertaining a control parameter (K) that represents a relationship between the venous volume flow ($Q_v$) through the venous fluid line (30) and the arterial volume flow ($Q_a$) through the arterial fluid line (20);
   ascertaining a theoretical control parameter (Kt) that represents a theoretical relationship between the venous volume flow ($Q_v$) and the arterial volume flow ($Q_a$), the theoretical control parameter (Kt) being ascertained as a function of the pump volume flow ($Q_p$) and a filtration volume flow ($Q_F$), which is led away from the blood chamber (11) via the semipermeable membrane (13);
   comparing the ascertained control parameter (K) to the ascertained theoretical control parameter ($K_t$);
   monitoring the operation on the basis of the comparison.

2. Method according to Claim 1, comprising the steps of:

   recording a change in the control parameter (K) over time (t);
   monitoring the operation on the basis of the recorded change of the control parameter (K) over time.

3. Method according to either of the preceding claims, wherein the arterial volume flow ($Q_a$) and the venous volume flow ($Q_v$) are each detected directly by measurement.

4. Method according to Claim 1 or 2, wherein the arterial volume flow ($Q_a$) and the venous volume flow ($Q_v$) are ascertained approximately on the basis of the Hagen-Poiseuille equation.

5. Method according to Claim 4, wherein the approximate ascertainment comprises:

   ascertaining a venous differential pressure ($P_v$-$P_m$) between the venous fluid line (30) and the venous vessel access ($Z_v$) and an arterial differential pressure ($P_m$-$P_a$) between the arterial fluid line (20) and the arterial vessel access ($Z_a$);
   ascertaining an arterial viscosity ($\eta_a$) of arterial volume flow ($Q_a$) and venous viscosity ($\eta_v$) of venous volume flow ($Q_v$);
   calculating the control parameter (K) as a function of the recorded differential pressures ($P_v$-$P_m$, $P_m$-$P_a$) and the recorded viscosities ($\eta_a$, $\eta_v$).

6. Method according to Claim 5, wherein the ascertainment of the differential pressures ($P_v$-$P_m$, $P_m$-$P_a$) comprises:

   measuring a venous fluid pressure ($P_v$) in the venous fluid line (30) and an arterial fluid pressure ($P_a$) in the arterial fluid line (20);
   recording, especially non-invasively measuring, a mean arterial pressure ($P_m$) of the patient.

7. Method according to Claim 5 or 6, wherein the ascertainment of the viscosities ($\eta_a$, $\eta_v$) comprises:

   measuring an arterial haematocrit value ($H_a$) of the blood in the arterial fluid line (20) and/or a venous haematocrit value ($H_v$) of the blood in the venous fluid line (30);
   ascertaining the arterial viscosity ($\eta_v$) as a function of the measured arterial haematocrit value ($H_a$) and/or venous viscosity ($\eta_v$) as a function of the measured venous haematocrit value ($H_v$).

8. Method according to Claim 7, wherein the arterial haematocrit value ($H_a$) is recorded by measurement and the venous viscosity ($\eta_v$) is ascertained in approximated fashion as a function of the pump volume flow ($Q_p$), the filtration volume flow ($Q_F$) and the arterial haematocrit value ($H_a$) recorded by measurement.

9. Method according to any of the preceding claims, wherein the operation of the device (1) is monitored over a plurality of temporally successive treatments of the patient, and control parameters ascertained in each case in the process are compared to one another, the operation being monitored in a manner dependent on the comparison.

10. Monitoring system (50) for a medical device (1) for extracorporeal blood treatment, the device (1) comprising a blood treatment unit (10) having a blood chamber (11) configured for traversal by blood and a treatment chamber (12) configured for traversal by a blood treatment fluid and separated from the blood chamber (11) by way of a semipermeable membrane (13),

an arterial fluid line (20) that is connected in fluid-conveying fashion at one end to an inlet (14) in the blood chamber (11), connectable in fluid-conveying fashion at the other end to an arterial vessel access ($Z_a$) of a patient and configured for traversal by an arterial volume flow ($Q_a$), a venous fluid line (30) that is connected in fluid-conveying fashion at one end to an outlet (15) from the blood chamber (11), connectable in fluid-conveying fashion at the other end to a venous vessel access ($Z_v$) of the patient and configured for traversal by a venous volume flow ($Q_v$), and a blood pump (40) that is configured to convey a pump volume flow ($Q_p$) by pumping through an extracorporeal blood circuit comprising the blood chamber (11), the arterial fluid line (20) and the venous fluid line (30), wherein the monitoring system (50) comprises:

an ascertainment means (60) that is configured for ascertaining a control parameter (K) that represents a relationship between the venous volume flow ($Q_v$) through the venous fluid line (30) and the arterial volume flow ($Q_a$) through the arterial fluid line (20); ascertaining a theoretical control parameter ($K_t$) that represents a theoretical relationship between the venous volume flow ($Q_v$) and the arterial volume flow ($Q_a$), the ascertainment means (60) being configured to ascertain the theoretical control parameter ($K_t$) as a function of the pump volume flow ($Q_p$) and a filtration volume flow ($Q_F$), which is led away from the blood chamber (11) via the semipermeable membrane (13); comparing the ascertained control parameter (K) to the ascertained theoretical control parameter ($K_t$); a monitoring means (70) that is configured for monitoring the operation of the medical device (1) on the basis of the comparison between the ascertained control parameter (K) and the ascertained theoretical control parameter ($K_t$).

11. Monitoring system (50) according to Claim 10, **characterized in that** a recording means (80) is present and comprises a venous pressure sensor (81) that is configured for ascertaining a venous fluid pressure ($P_v$) in the venous fluid line (30), an arterial pressure sensor (82) that is configured for ascertaining an arterial fluid pressure ($P_a$) in the arterial fluid line (20), a blood pressure measuring apparatus (83) that is configured for recording a mean arterial pressure ($P_m$) of the patient and at least one haematocrit sensor (84) that is configured for recording an arterial or venous haematocrit value ($H_a$, $H_v$) in the respective fluid line (20, 30), wherein the ascertainment means (60) is configured for ascertaining the control parameter (K) as a function of the quantities recorded by means of the recording means (80).

12. Monitoring system (50) according to Claim 10 or 11, **characterized in that** the monitoring means (70) is configured for acoustic, optical and/or data-based output of at least one monitoring signal (S).

13. Monitoring system (50) according to any of Claims 10 to 12, wherein the monitoring system (50) is formed by components of the medical device (1).

14. Medical device (1) for extracorporeal blood treatment, comprising

a blood treatment unit (10) having a blood chamber (11) configured for traversal by blood and a treatment chamber (12) configured for traversal by a blood treatment fluid and separated from the blood chamber (11) by way of a semipermeable membrane (13), an arterial fluid line (20) that is connected in fluid-conveying fashion at one end to an inlet (14) in the blood chamber (11), connectable in fluid-conveying fashion at the other end to an arterial vessel access ($Z_a$) of a patient and configured for traversal by an arterial volume flow ($Q_a$), a venous fluid line (30) that is connected in fluid-conveying fashion at one end to an outlet (15) from the blood chamber (11), connectable in fluid-conveying fashion at the other end to a venous vessel access ($Z_v$) of the patient and configured for traversal by a venous volume flow ($Q_v$), and a blood pump (40) that is configured to convey a pump volume flow ($Q_p$) by pumping through an extracorporeal blood circuit comprising the blood chamber (11), the arterial fluid line (20) and the venous fluid line (30), and comprising a monitoring system (50) according to any of Claims 10 to 13.

**Revendications**

1. Procédé de surveillance du fonctionnement d'un appareil médical (1) pour le traitement extracorporel du sang, l'appareil (1) comprenant

une unité de traitement du sang (10) comprenant une chambre à sang (11) traversée par le sang, et une chambre de traitement (12) traversée par un liquide de traitement du sang et séparée de la chambre à sang (11) par une membrane semi-perméable (13), une conduite de fluide artérielle (20) reliée de manière fluidique à une entrée (14) de la chambre à sang (11) à une extrémité et à un accès vasculaire artériel ($Z_a$) d'un patient à l'autre extrémité, et traversée par un flux volumique artériel ($Q_a$), une conduite de fluide veineuse (30) reliée de manière fluidique à une sortie (15) de la chambre à sang (11) à une extrémité et à un accès vasculaire veineux ($Z_v$) du patient à l'autre extrémité, et traversée par un flux volumique veineux ($Q_v$), et une pompe à sang (40) conçue pour faire circuler un flux volumique de pompe ($Q_p$) à travers une circulation sanguine extracorporelle qui comprend la chambre à sang (11), la conduite de fluide artérielle (20) et la conduite de fluide veineuse (30),

le procédé comprenant les étapes suivantes :

déterminer un paramètre de contrôle (K) représentant un rapport entre le flux volumique veineux ($Q_v$) à travers la conduite de fluide veineuse (30) et le flux volumique artériel ($Q_a$) à travers la conduite de fluide artérielle (20) ; déterminer un paramètre de contrôle théorique (Kt) représentant un rapport théorique entre le flux volumique veineux ($Q_v$) et le flux volumique artériel ($Q_a$), le paramètre de contrôle théorique (Kt) étant déterminé en fonction du flux volumique de pompe ($Q_p$) et

d'un flux volumique de filtration ($Q_F$) qui est dérivé de la chambre à sang (11) à travers la membrane semi-perméable (13) ; comparer le paramètre de contrôle (K) déterminé au paramètre de contrôle théorique (Kt) déterminé ; surveiller le fonctionnement en fonction de la comparaison.

2. Procédé selon la revendication 1, comprenant les étapes suivantes :

détecter une variation du paramètre de contrôle (K) au cours du temps (t) ; surveiller le fonctionnement en fonction de la variation temporelle détectée du paramètre de contrôle (K).

3. Procédé selon l'une quelconque des revendications précédentes, dans lequel le flux volumique artériel ($Q_a$) et le flux volumique veineux ($Q_v$) sont chacun détectés par une technique de mesure directe.

4. Procédé selon la revendication 1 ou 2, dans lequel le flux volumique artériel ($Q_a$) et le flux volumique veineux ($Q_v$) sont déterminés de manière approximative sur la base de l'équation de Hagen-Poiseuille.

5. Procédé selon la revendication 4, dans lequel la détermination approximative comprend :

la détermination d'une pression différentielle veineuse ($P_v$-$P_m$) entre la conduite de fluide veineuse (30) et l'accès vasculaire veineux ($Z_v$) et d'une pression différentielle artérielle ($P_m$-$P_a$) entre la conduite de fluide artérielle (20) et l'accès vasculaire artériel ($Z_a$) ; la détermination d'une viscosité artérielle ($\eta_a$) du flux volumique artériel ($Q_a$) et d'une viscosité veineuse ($\eta_v$) du flux volumique veineux ($Q_v$) ; le calcul du paramètre de contrôle (K) en fonction des pressions différentielles ($P_v$-$P_m$, $P_m$-$P_a$) détectées et des viscosités ($\eta_a$, $\eta_v$) détectées.

6. Procédé selon la revendication 5, dans lequel la détermination des pressions différentielles ($P_v$-$P_m$, $P_m$-$P_a$) comprend :

la mesure d'une pression de fluide veineuse ($P_v$) dans la conduite de fluide veineuse (30) et d'une pression de fluide artérielle ($P_a$) dans la conduite de fluide artérielle (20) ; la détection, notamment la mesure non invasive, d'une pression artérielle moyenne ($P_m$) du patient.

7. Procédé selon la revendication 5 ou 6, dans lequel la détermination des viscosités ($\eta_a$, $\eta_v$) comprend :

la mesure d'une valeur d'hématocrite artérielle ($H_a$) du sang dans la conduite de fluide artérielle (20) et/ou d'une valeur d'hématocrite veineuse ($H_v$) du sang dans la conduite de fluide veineuse (30) ;

la détermination de la viscosité artérielle ($\eta_v$) en fonction de la valeur d'hématocrite artérielle mesurée ($H_a$) et/ou de la viscosité veineuse ($\eta_v$) en fonction de la valeur d'hématocrite veineuse mesurée ($H_v$).

8. Procédé selon la revendication 7, dans lequel la valeur d'hématocrite artérielle ($H_a$) est détectée par une technique de mesure et la viscosité veineuse ($\eta_v$) est déterminée de manière approximative en fonction du flux volumique de pompe ($Q_p$), du flux volumique de filtration ($Q_F$) et de la valeur d'hématocrite artérielle détectée ($H_a$).

9. Procédé selon l'une quelconque des revendications précédentes, dans lequel le fonctionnement de l'appareil (1) est surveillé lors de plusieurs traitements successifs du patient, et les paramètres de contrôle déterminés sont comparés les uns aux autres, le fonctionnement étant surveillé en fonction de la comparaison.

10. Système de surveillance (50) destiné à un appareil médical (1) pour le traitement extracorporel du sang, l'appareil (1) comprenant

une unité de traitement du sang (10) comprenant une chambre à sang (11) conçue pour être traversée par le sang, et une chambre de traitement (12) conçue pour être traversée par un liquide de traitement et séparée de la chambre à sang (11) par une membrane semi-perméable (13), une conduite de fluide artérielle (20) reliée de manière fluidique à une entrée (14) de la chambre à sang (11) à une extrémité, pouvant être reliée de manière fluidique à un accès vasculaire artériel ($Z_a$) d'un patient à l'autre extrémité, et conçue pour être traversée par un flux volumique artériel ($Q_a$),
une conduite de fluide veineuse (30) reliée de manière fluidique à une sortie (15) de la chambre à sang (11) à une extrémité, pouvant être reliée de manière fluidique à un accès vasculaire veineux ($Z_v$) du patient à l'autre extrémité, et conçue pour être traversée par un flux volumique veineux ($Q_v$), et
une pompe à sang (40) conçue pour pomper un flux volumique de pompe ($Q_p$) à travers une circulation sanguine extracorporelle comprenant la chambre à sang (11), la conduite de fluide artérielle (20) et la conduite de fluide veineuse (30),
le système de surveillance (50) comprenant :

un dispositif de détermination (60) conçu pour déterminer un paramètre de contrôle (K) représentant un rapport entre le flux volumique veineux ($Q_v$) à travers la conduite de fluide veineuse (30) et le flux volumique artériel ($Q_a$) à travers la conduite de fluide artérielle (20) ;

déterminer un paramètre de contrôle théorique (Kt) représentant un rapport théorique entre le flux volumique veineux ($Q_v$) et le flux volumique artériel ($Q_a$), le dispositif de détermination (60) étant conçu pour déterminer le paramètre de contrôle théorique (Kt) en fonction du flux volumique de pompe ($Q_p$) et d'un flux volumique de filtration ($Q_F$) qui est dérivé de la chambre à sang (11) à travers la membrane semi-perméable (13) ;
comparer le paramètre de contrôle (K) déterminé au paramètre de contrôle théorique (Kt) déterminé ;
un dispositif de surveillance (70) conçu pour surveiller le fonctionnement de l'appareil médical (1) en fonction de la comparaison entre le paramètre de contrôle (K) déterminé et le paramètre de contrôle théorique ($K_t$) déterminé.

11. Système de surveillance (50) selon la revendication 10, **caractérisé en ce qu'**il comprend un dispositif de détection (80) comportant un capteur de pression veineuse (81) conçu pour détecter une pression de fluide veineuse ($P_v$) dans la conduite de fluide veineuse (30), un capteur de pression artérielle (82) conçu pour détecter une pression de fluide artérielle ($P_a$) dans la conduite de fluide artérielle (20), un dispositif de mesure de pression sanguine (83) conçu pour détecter une pression artérielle moyenne ($P_m$) du patient, et au moins un capteur d'hématocrite (84) conçu pour détecter une valeur d'hématocrite artérielle ou veineuse ($H_a$, $H_v$) dans la conduite de fluide (20, 30) respective, le dispositif de détermination (60) étant conçu pour déterminer le paramètre de contrôle (K) en fonction des grandeurs détectées au moyen du dispositif de détection (80).

12. Système de surveillance (50) selon la revendication 10 ou 11, **caractérisé en ce que** le dispositif de surveillance (70) est conçu pour délivrer au moins un signal de surveillance (S) de manière acoustique, optique et/ou à base de données.

13. Système de surveillance (50) selon l'une quelconque des revendications 10 à 12, dans lequel le système de surveillance (50) est formé par des composants de l'appareil médical (1).

14. Appareil médical (1) pour le traitement extracorporel du sang, comprenant

une unité de traitement du sang (10) comprenant une chambre à sang (11) conçue pour être traversée par le sang, et une chambre de traitement (12) conçue pour être traversée par un liquide de traitement et séparée de la chambre à sang (11) par une membrane semi-perméable (13), une conduite de fluide artérielle (20) reliée de manière fluidique à une entrée (14) de la chambre à sang (11) à une extrémité, pouvant être reliée de manière fluidique à un accès vasculaire artériel ($Z_a$) d'un patient à l'autre extrémité, et conçue pour être traversée par un flux volumique artériel ($Q_a$),

une conduite de fluide veineuse (30) reliée de manière fluidique à une sortie (15) de la chambre à sang (11) à une extrémité, pouvant être reliée de manière fluidique à un accès vasculaire veineux ($Z_v$) du patient à l'autre extrémité, et conçue pour être traversée par un flux volumique veineux ($Q_v$), et

une pompe à sang (40) conçue pour pomper un flux volumique de pompe ($Q_p$) à travers une circulation sanguine extracorporelle comprenant la chambre à sang (11), la conduite de fluide artérielle (20) et la conduite de fluide veineuse (30),

et comprenant un système de surveillance (50) selon l'une quelconque des revendications 10 à 13.

FIG. 1

FIG. 2

FIG. 3

FIG. 4

FIG. 5

FIG. 6

**IN DER BESCHREIBUNG AUFGEFÜHRTE DOKUMENTE**

**In der Beschreibung aufgeführte Patentdokumente**

- EP 2913072 A1
- EP 1938847 A2